# EUROPEAN PATENT SPECIFICATION

(11) **EP 0 430 066 B1**
(45) Date of publication and mention of the grant of the patent: **31.08.1994**
(21) Application number: 90122299.2
(22) Date of filing: 22.11.1990
(51) Int. Cl.: C12N 15/32, C12N 15/31, C12N 15/65, C12N 1/20, C12Q 1/68

(54) **DNA-sequence conferring resistance to the antibiotic thermorubin**
Thermorubin-Antibiotikum widerstandsfähigkeitstragende DNA-Sequenz
Séquence d'ADN conférant la résistance à l'antibiotique thermorubin

(30) Priority: 28.11.1989 EP 89203015
(43) Date of publication of application: 05.06.1991
(73) Proprietor: GRUPPO LEPETIT S.p.A., I-20020 Lainate (MI) (IT)
(72) Inventor: Denaro, Maurizio, I-20090 Opera (Milano) (IT); Lorenzetti, Rolando, I-20052 Monza (Milano) (IT)
(74) Representative: Sgarbi, Renato

(56) References cited:
- BIOCHEMISTRY. vol. 21, 1982, EASTON, PA US pages 484 - 491; LIN F-L et al: "Theprotein synthesis inhibitor thermorubin.2.Mechanism of inhibition of initiationon Escherichia coli ribosomes."

## Description

The present invention relates to a new DNA fragment or a sub-fragment thereof which is capable of conferring resistance to thermorubin (hereinbelow: TRB), upon transformation into a suitable microbial host by a proper vector.

Thermorubin is a polyketide antibiotic substance produced by Thermoactinomyces antibioticus ATCC 14570 which is described in US Patent 3300379 and J.Am.Chem.Soc. (1972) 94, 3269-72. It is known to possess good in vitro antibacterial activity.

One object of the present invention is a DNA sequence capable of conferring a resistance to TRB when used to transform a sensitive host by means of a suitable vector.

A mechanism for such a resistance is not yet definitely proven, anyway, the present disclosure is not intended as limited to any mechanism of action or theory on it.

For convenience, a DNA sequence of the invention, as mentioned above, will be referred to as "TRB-R conferring sequence".

An example of such TRB-R conferring sequence is contained in a Sau 3AI fragment of about 4.0 Kbp obtainable as a restriction fragment from the genome of Thermoactinomyces antibioticus ATCC 14570.

Thermoactinomyces antibioticus ATCC 14570 is a publicly available strain which can be obtained on request from the American Type Culture Collection, Rockville, Maryland 20852, U.S.A. (see ATCC Catalogue of Bacteria & Bacteriophages, 17th Edition, 1989, page 249).

A DNA fragment of the invention is thus a DNA fragment of about 4.0 Kbp which can be obtained by Sau 3AI treatment of the genome of Thermoactinomyces antibioticus ATCC 14570, (or a DNA sequence containing it), a sub-fragment thereof, which at medium-low stringency hybridizes to a significant extent with the above fragment, or with a probe derivable from it, and is capable of conferring a resistance to TRB, upon transformation into a sensitive microbial host. Therefore, the invention encompasses also DNA sequences which hybridize under high stringency with the above mentioned restriction fragment, or a sub-fragment thereof, and maintain the ability to confer a resistance to TRB, upon transformation of a sensitive host.

All the above DNA sequences are encompassed by the definition of "TRB-R conferring sequence", which therefore includes:
a) a DNA fragment of about 4.0 Kbp which can be obtained by Sau 3AI treatment of the genome of Thermoactinomyces antibioticus ATCC 14570, a sub-fragment thereof or a DNA sequence containing it, which is capable of conferring a resistance to TRB upon transformation into a sensitive microbial host;
b) a nucleic acid sequence which is capable of conferring a resistance to TRB upon transformation into a sensitive microbial host, and hybridize with a DNA fragment mentioned above under a);
c) portions of DNA inserts hybridizing with the fragments mentioned under a), and capable of conferring a TRB-R-mediated resistance;
d) DNA sequences which are degenerated as a result of the degenerated genetic code to DNA sequences defined under a) or b) and are capable of conferring a resistance to TRB upon transformation into a sensitive microbial host.

A TRB-R conferring sequence can be used in constructing a vector that confers the TRB-R mediated-resistance to TRB upon transformation of a sensitive microbial host.

The following definitions relate to terms and expressions that are used in this description. They are commonly understood and used in the art, but are reported here for convenience:
"A Recombinant DNA Cloning Vector", or "Vector" (for conciseness), is any autonomously replicating or integrating agent, including, but not limited to plasmids, cosmids, phages, etc.

A "Restriction Fragment" is any linear DNA sequence obtained by digesting a DNA with one or more restriction enzymes.

A "Nucleic Acid Sequence" is a polynucleotide sequence of natural, synthetic or hybrid origin, either of DNA or RNA.

A "Sensitive Microbial Host" is a host cell that cannot grow in the presence of TRB, unless it is transformed with a TRB-R conferring sequence of the invention.

"Transformant" is a recipient host cell that has undergone transformation, while "Transformation" is the introduction of DNA into a recipient host cell that changes the genotype and results in a change in the recipient cell.

The "Host Cell" may be any of the known prokariotic or eukariotic microorganisms for which a transforming vector is known or can be devised on the basis of the knowledge in the art. Thus, it includes cells of mammalian, avian, amphibian, yeast, bacterial and vegetal origin. A preferred group of hosts is represented by bacteria such as E.coli K 12 or Thermoactinomyces antibioticus.

One of the most preferred groups of microbial hosts is represented by the microorganisms which produce TRB, such as the Thermoactinomyces strain reported above.

With the expression "TRB-R-Mediated Resistance" it is intended the resistance to thermorubin antibiotic produced by transformation of a sensitive microbial host with a TRB-R conferring sequence.

With the term "Hybridization" the hybridization of nucleic acids is intended, i.e. the process whereby two single-stranded polynucleotides form a double-stranded molecule, with hydrogen bonding between complementary bases in the two strands. Hybridization can take place between complementary strands of both DNA and RNA to produce duplex DNA, duplex RNA or duplex DNA-RNA hybrid molecules. This process makes it possible to identify specific DNA sequences by hybridization with tagged DNA or RNA probes. The conditions of the hybridization are sometimes qualified as "low", "high" or "medium" stringency depending on the concentration of the buffered saline employed, that is generally a sodium chloride/sodium citrate solution (conventionally, SSC).
In particular, "medium-low stringency" refers to concentrations of this solution in the range of 1 x SSC (i.e. a solution of 0.15 M sodium chloride and 0.015 M sodium citrate) or more, while "high stringency" refers to concentrations in the range of about 0.1-0.5 x SSC.

### Detailed description of the invention

A TRB-R conferring sequence of the invention can be used to confer a selectable resistance to TRB upon transformation of a sensitive microbial host with a suitable TRB-R containing vector.
For example, a plasmid vector for E. coli (such as pUC18) carrying the Ampicillin resistance gene can be used to insert a TRB-R conferring sequence in a appropriate restriction site to obtain a plasmid with two resistance markers: Ampicillin (Amp) and TRB (TRB-R). The new plasmid can then be linearized by digestion with a restriction enzyme which possesses at least one recognition site inside the TRB resistance gene (TRB-R), thus leaving the origin of replication and the resistance to Ampicillin unaltered. The insertion of a foreign DNA in its TRB-R region renders the transformed organism selectable for the simultaneous resistance to Ampicillin and sensitivity to TRB.

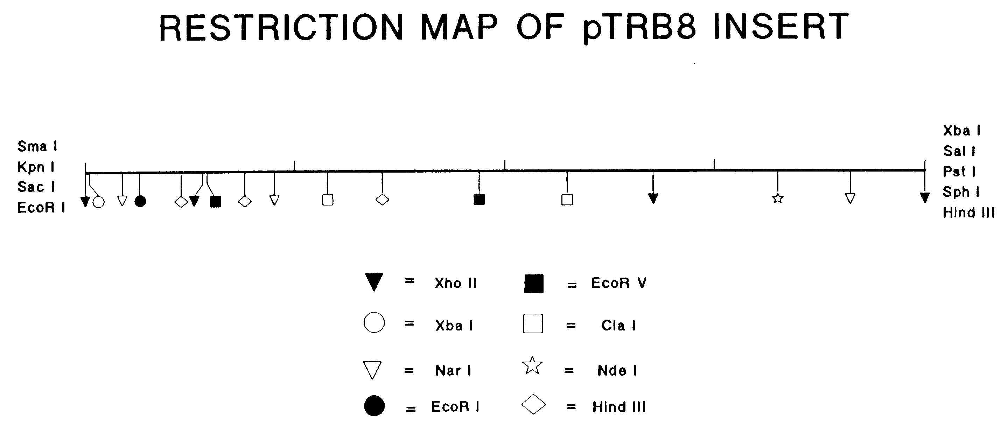

If the TRB-R conferring sequence is a partial Sau 3AI restriction fragment described above, internal restriction sites that can be conveniently used are the ClaI and/or EcoRV restriction sites (c.f. Fig. 1).

According to another embodiment of the invention, a TRB-R coding sequence can be introduced, via an appropriate vector, into a Thermoactinomyces antibioticus ATCC 14570 strain or into any other TRB-producing strain. This transformation of a producer strain may improve the antibiotic production yields by conferring, or more likely, by increasing, the degree of resistance of the strain to its antimicrobial product. In many instances in fact, the efficiency of a microbiological process for producing an antibiotic substance is limited by the sensitivity of the producer to high concentrations of its own product (c.f. Katagiri K., J. Antibiotics, 7, 45-52, 1954).

Another application of a sequence of the invention is represented by its use as a probe to detect the presence of similar DNA sequences in clinical isolates under medium-low stringency conditions. For this purpose, a probe represented by a tagged TRB-R sequence is hybridized with a nucleic acid fraction of the test sample. The positivity to this test might indicate the presence of this kind of resistance in the isolates, thus suggesting the possibility that the tested strains can develop, or have already developed, a TRB-R type of resistance against thermorubin.

A TRB-R conferring sequence of the invention can be prepared from a DNA extract of Thermoactinomyces antibioticus ATCC 14570 after partial digestion with a specific restriction enzyme (e.g. Sau 3AI), ligation to a known vector for a given host cell which is naturally sensitive to TRB, selection of the transformed cells which have acquired a resistance to TRB and isolation of the DNA sequence which is responsible for the acquired resistance. The single process steps are performed according to techniques known per se in the art that need not be discussed in great details here, since they are readily repeatable by the skilled technician on the basis of the information contained in the present disclosure.

Other methods for preparing a TRB-R conferring sequence of the invention are apparent to the skilled technician, also in view of the present disclosure and include using a sequence such as the above fragment of Thermoactinomyces antibioticus ATCC 14570 or a subfragment thereof, to fish out DNA sequences that hybridize with it at medium-low stringency (or high stringency) and possess the TRB-R conferring capability. Also synthetic probes can be prepared by the conventional methods after sequencing a TRB-R-conferring sequence or a portion thereof, e.g. specifically, the gene coding for the TRB resistance.

Other processes for preparing a TRB-R conferring sequence include copying a RNA sequence into a cDNA which is then used to confer the TRB-R-mediated resistance, according to the usual transformation techniques or similarly known techniques.

Many of the molecular biology methodologies and protocols reported or referred to in the following sections are known per se in the art and are part of the background knowledge of a person of ordinary skill in this art. They are reported also in many reference books and manuals such as: Hopwood D.A. et al., (1985) Genetic Manipulation of Streptomyces, a Laboratory Manual, The John Innes Foundation, Norwich, U.K; Maniatis T. et al. (1982), Molecular Cloning, a Laboratory Manual, C.S.H. Laboratory, Cold Spring Harbour, N.Y; and Current Protocols in Molecular Biology, 1987, Greene Publishing Associates and Wiley-Interscience, N.Y.

To avoid lengthy repetitions of these known techniques that are boring, time-consuming and superfluous to the skilled reader, in this specification extensive resort is made to citations of a given reference book or manual, both for known protocols and methodologies. For conciseness, the above mentioned manuals will be referred to respectively as "Hopwood", "Maniatis" and "Current Protocols".

### Brief description of the drawings

Fig. 1 Partial restriction map of the Sau 3AI DNA fragment of about 4.0 Kbp of the genomic DNA of Thermoactinomyces antibioticus ATCC 14570. The approximate number and positions of the recognition sites were determined by single and double digestions with the appropriate restriction enzymes, the dimensions of the resulting DNA fragments were determined by gel electrophoresis.
   The map reveals an insert containing two Xho II sites at its ends, (about 200 bp), which were originated by joining Sau 3AI and BamHI sticky ends, and internal restriction sites at least for ClaI, EcoRI, EcoRV, HindIII, NarI, NdeI, XbaI, XhoII.
   Recognition sites for the following restriction enzymes were not detected: BamHI, BglII, KpnI, PstI, SacI, SalI, SmaI, SphI.
   The restriction sites of the multiple cloning site of pUC18 are reported on the borders.

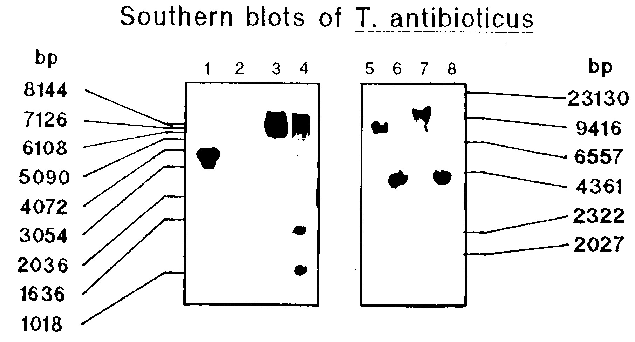

Fig. 2 shows the results of hydridization experiments (Southern blot) with Thermoactinomyces antibioticus ATCC 14570 genomic DNA. The EcoRI-SalI fragment of plasmid pTRB8 was used as probe in the Southern hydridizations. The genomic DNA was subjected to restriction enzyme digestions, followed by electrophoretic separation of the restricted DNA, and then transferred onto a nylon membrane. The digoxygenin-labelled probe was hybridized with the genomic DNA from Thermoactinomyces antibioticus ATCC 14570 at 68°C for 16 h. Excess probe was washed away with 15 mM NaCl and 1.5 mM sodium citrate at the same temperature. Lane 1 contained the probe, as a positive control. Lane 2 contained the lKbp DNA ladder (GIBCO-BRL catalogue 1988, 520-5615) as a negative control. The restricted genomic DNA was loaded in the other lanes as follows: lane 3: BamHI; lane 4: ClaI; lane 5: BglII; lane 6: EcoRI, lane 7: PstI; lane 8: PvuII. The M.W. markers are indicated on the left and right sides.
   Lanes 1-4 are from a shorter electrophoretic separation to evidence shorter DNA fragments (the corresponding M.W. markers are on the left; lanes 5-8 are from a longer run and the corresponding M.W. markers are, in this case, on the right side).

The following examples are intended as an illustration of the invention and of the way in which it can be practiced and, as such, cannot be construed as imposing any limitation to its scope.

### Example 1:

### Isolation of a TRB-R conferring DNA sequence

1.1 The total genomic DNA of Thermoactinomyces antibioticus ATCC 14570 was isolated, partially digested with the restriction endonuclease Sau 3AI and size fractionated by Agarose gel electrophoresis according to the procedures described in Hopwood (see pages 79-80, in particular) and Current Protocols (see in particular Section 2, unit 2.6 and specifically 2.6.1 to 2.6.8).
1.2 The 3.5 to 10 Kb fragments are then ligated with BamHI linearized, phosphatase-treated plasmid pUC18 (commercially available; its restriction map is reported in Current Protocols 1.5.4). This ligation step is carried out essentially according to Hopwood (pages 154-157); the linearization and phosphatase-treatment of pUC18 are carried out following the usual procedures, c.f. Hopwood pages 131-135, 158-159 and 164.
1.3 The obtained ligation mixture is then used to transform E. coli cells (see for instance Hopwood pages 31-32 and 120-121 for further technical or methodological details). After selection with Ampicillin (50 mg/l), about 3600 transformants were obtained which contained recombinant plasmids.
1.4 These transformants were then replicated on M 9 (Current Protocols 1.1.1-1.1.2) minimal plates containing 5 mg/l of thiamine, 0.1 % Casamino acids and 25 mg/l of TRB to select for TRB-resistance; 9 clones were selected for their ability to grow in the presence of the antibiotic. By restriction mapping they appeared to be all the same. The plasmid present in these clones was named pTRB8.

### Example 2:

### Confirmation that the selected plasmid contains the TRB-R conferring sequence

2.1 Plasmid pTRB8 is extracted (Current Protocols 1.6.1-1.6.6) from the above obtained cells and used to retransform E. coli DH5alpha competent cells (according to the methodology described in Example 1.3). All the Ampicillin-resistant transformants were also resistant to TRB, thus demonstrating that the TRB resistance was linked to pTRB8.
2.2 The restriction map of the plasmid revealed an insert about 4.0 Kbp long (c.f. Fig. 1) containing two Xho II sites at its ends, which were originated by joining Sau 3AI and BamHI sticky ends, restriction sites for ClaI, EcoRI, EcoRV, HindIII, NarI, NdeI, XbaI, XhoII and apparently no recognition sites for BamHI, BglII, KpnI, PstI, SacI, SalI, SmaI, SphI.
2.3 A major portion of the insert was isolated by digestion with EcoRI and SalI, Digoxygenin-labelled and used in Southern blot hybridizations with the genomic DNA of Thermoactinomyces antibioticus ATCC 14570 digested in turn with various restriction enzymes (BamHI, BglII, EcoRI, PstI, PvuII and ClaI) according to the known procedures. Hybridizations are conducted in the conventional way, as reported also in the instruction of the labelling kit manufacturer (Biochemia Boehringer Mannheim, DNA Labelling and Detection, Non Radioactive, 1989). Labelling of the probe is made with Digoxygenin-11-dUTP (Boehringer Mannheim Biochemia), while the genomic DNA, after restriction according to the procedure already reported above, is electrophoretically separated on 1% agarose and transferred onto a nylon membrane (Gene Screen Plus; New England Nuclear). The gel containing the separated DNA bands is incubated in 0.4 N NaOH-0.6 M NaCl for 30 min at room temperature to denature the DNA, then it is incubated with 1.5 M NaCl-0.5 M TRIS-HCl pH 7.5 for 30 min and transferred onto a nylon membrane (Gene Screen Plus, NEN) prepared in 10 x SSC (1.5 M NaCl-0.15 M sodium citrate). The transfer is allowed to continue overnight in 10XSSC, then the membrane is air-dried and UV treated. The hybridization with the tagged probe is conducted at 68°C according to the known procedures which include a pre-hybridization with 10 ml of a solution of 5 x SSC, 0.1% (w/v) N-laurylsarcosine, 0.2% (w/v) SDS (sodium dodecyl sulfate), and 1% Blocking Reagent (Boehringer Mannheim, DNA Labelling and Detection Non Radioactive Kit No. 1093657 vial 11) in a sealable plastic bag which is then sealed and incubated for about 1 hour at 68°C. Then, the pre-hybridization solution is discarded and about 2.5 ml of fresh pre-hybridization solution is put on the membrane along with a solution of denaturated tagged probe (final concentration of about 10 ng/ml). The resealed bag is incubated for about 16 h at 68°C. Excess probe is washed away with 0.1 x SSC and 0.1% SDS, at 68°C.
   The results obtained are consistent with the hypothesis that the insert in pTRB8 is present as a unique sequence in the Thermoactinomyces antibioticus ATCC 14570 genome.

## Claims

1. A TRB-R conferring sequence which comprises a nucleotidic sequence selected from:
a) a DNA fragment of about 4.0 Kbp which can be obtained by Sau 3AI treatment of the genome of Thermoactinomyces antibioticus ATCC 14570, a subfragment thereof or a DNA sequence containing it, which is capable of conferring a resistance to TRB upon transformation into a sensitive microbial host;
b) a nucleic acid sequence which is capable of conferring a resistance to TRB upon transformation into a sensitive microbial host, and hybridize with a DNA fragment mentioned above under a);
c) portions of DNA inserts hybridizing with the fragments mentioned under a), and capable of conferring a TRB-R-mediated resistance;
d) DNA sequences which are degenerated as a result of the degenerated genetic code to DNA sequences defined under a) or b) and are capable of conferring a resistance to TRB upon transformation into a sensitive microbial host.

2. A TRB-R conferring sequence which is a DNA fragment of about 4.0 Kbp which can be obtained by Sau 3AI treatment of the genome of Thermoactinomyces antibioticus ATCC 14570, a subfragment thereof or a DNA sequence containing it, which is capable of conferring a resistance to TRB upon transformation into a sensitive microbial host.

3. A TRB-R conferring sequence which is a nucleic acid sequence which is capable of conferring a resistance to TRB upon introduction into a sensitive microbial host, and hydridizes with a DNA fragment of about 4.0 Kbp which can be obtained by Sau 3AI treatment of the genome of Thermoactinomyces antibioticus ATCC 14570, a subfragment thereof or a DNA sequence containing it.

4. A TRB-R conferring sequence which is a portion of a DNA insert hybridizing with a DNA fragment of about 4.0 Kbp which can be obtained by Sau 3AI treatment of the genome of Thermoactinomyces antibioticus ATCC 14570, a subfragment thereof or a DNA sequence containing it, which is capable of conferring a resistance to TRB upon transformation into a sensitive microbial host.

5. A recombinant plasmid suited for transformation of a microbial host comprising a TRB-R conferring sequence of claims 1, 2, 3 or 4.

6. A microbial host cell transformed with a recombinant plasmid of claim 5.

7. A microbial cell according to claim 6 which is a producer of thermorubin.

8. A process for preparing a TRB-R conferring sequence selected from:
a) a DNA fragment of about 4.0 Kbp which can be obtained by Sau 3AI treatment of the genome of Thermoactinomyces antibioticus ATCC 14570, a subfragment thereof or a DNA sequence containing it, which is capable of conferring a resistance to TRB upon transformation into a sensitive microbial host;
b) a nucleic acid sequence which is capable of conferring a resistance to TRB upon transformation into a sensitive microbial host, and hybridizes with a DNA fragment mentioned above under a);
c) portions of DNA inserts hybridizing with the fragments mentioned under a), and capable of conferring a TRB-R-mediated resistance;
d) DNA sequences which are degenerated as a result of the degenerated genetic code to DNA sequences defined under a) or b) and are capable of conferring a resistance to TRB upon transformation into a sensitive microbial host
which comprises:
a) extracting the DNA fraction from the genome of a microorganism which is a TRB producer, and partially digesting it with a specific restriction enzyme
b) ligating the restriction fragments to a vector for a sensitive host cell
c) selecting the transformed cells by means of the acquired resistance and isolating the TRB-R conferring sequence therefrom.

9. A process as in claim 8 wherein the microorganism is Thermoactinomyces antibioticus ATCC 14570.

10. A process as in any one of claim 8 and 9 wherein the restriction enzyme of step a) is Sau 3AI.

11. A process as in any one of claims 8 to 10 wherein the restriction fragment is a DNA fragment of about 4.0 Kbp and the vector is BamHI linearized and phosphatase treated plasmid pUC18.

12. A process as in any one of claims 8 to 11 wherein the sensitive host cell is E. coli.

13. Use of a TRB-R conferring sequence of claims 1, 2, 3 or 4 to detect, isolate, or purify the corresponding regions, or inserts containing them, from the genome of a microbial cell.

14. Use according to claim 13 wherein the microbial cell is a TRB producer.

15. Use of a TRB-R conferring sequence of claims 1, 2, 3 or 4 to introduce or increase the resistance of a microbial host to TRB.

16. Use according to claim 15 wherein the microbial cell is a TRB producer.

17. A process for isolating, identifying or purifying a nucleic sequence capable of conferring a resistance to TRB, or an insert containing it, which comprises hybridizing a probe represented by a tagged TRB-R sequence according to claims 1, 2, 3 or 4 with a nucleic acid fraction, and optionally isolating the nucleic acid fragments that hybridize with the probe.

## Patentansprüche

1. TRB-R-verleihende Sequenz, umfassend eine Nucleotidsequenz ausgewählt aus:
a) einem DNA-Fragment von etwa 4,0 Kbp, das durch Sau 3AI-Behandlung des Genoms von Thermoactinomyces antibioticus ATCC 14570 erhalten werden kann, einem Subfragment davon oder einer dieses enthaltenden DNA-Sequenz, die fähig ist, nach Transformation in einen sensitiven mikrobiellen Wirt eine Resistenz gegenüber TRB zu verleihen;
b) einer Nucleinsäuresequenz, die fähig ist, nach Transformation in einen sensitiven mikrobiellen Wirt Resistenz gegenüber TRB zu verleihen, und mit einem vorstehend unter a) erwähnten DNA-Fragment hybridisiert;
c) Teilen von DNA-Insertionen, die mit den unter a) erwähnten Fragmenten hybridisieren und fähig sind, eine TRB-R-vermittelte Resistenz zu verleihen;
d) DNA-Sequenzen, die als Ergebnis der Degeneration des genetischen Codes zu DNA-Sequenzen degeneriert sind, die unter a) oder b) definiert sind und fähig sind, nach Transformation in einen sensitiven mikrobiellen Wirt Resistenz gegenüber TRB zu verleihen.

2. TRB-R-verleihende Sequenz, die ein DNA-Fragment von etwa 4,0 Kbp ist, das durch Sau 3AI-Behandlung des Genoms von Thermoactinomyces antibioticus ATCC 14570 erhalten werden kann, ein Subfragment davon oder eine dieses enthaltende DNA-Sequenz, die fähig ist, nach Transformation in einen sensitiven mikrobiellen Wirt Resistenz gegenüber TRB zu verleihen.

3. TRB-R-verleihende Sequenz, die eine Nucleinsäuresequenz ist, die fähig ist, nach Einführung in einen mikrobiellen sensitiven Wirt Resistenz gegenüber TRB zu verleihen, und mit einem DNA-Fragment von etwa 4,0 Kbp hybridisiert, das durch Sau 3AI-Behandlung des Genoms von Thermoactinomyces antibioticus ATCC 14570 erhalten werden kann, ein Subfragment davon oder eine dieses enthaltende DNA-Sequenz.

4. TRB-R-verleihende Sequenz, die ein Teil einer DNA-Insertion ist, die mit einem DNA-Fragment von etwa 4,0 Kbp hybridisiert, welches durch Sau 3AI-Behandlung des Genoms von Thermoactinomyces antibioticus ATCC 14570 erhalten werden kann, ein Subfragment davon oder eine dieses enthaltende DNA-Sequenz, die fähig ist, nach Transformation in einen sensitiven mikrobiellen Wirt Resistenz gegenüber TRB zu verleihen.

5. Rekombinantes Plasmid, das für die Transformation eines mikrobiellen Wirtes geeignet ist, umfassend eine TRB-R-verleihende Sequenz nach den Ansprüchen 1, 2, 3 oder 4.

6. Mikrobielle Wirtszelle, transformiert mit einem rekombinanten Plasmid nach Anspruch 5.

7. Mikrobielle Zelle nach Anspruch 6, die Thermorubin produziert.

8. Verfahren zur Herstellung einer TRB-R-verleihenden Sequenz, ausgewählt aus:
a) einem DNA-Fragment von etwa 4,0 Kbp, das durch Sau 3AI-Behandlung des Genoms von Thermoactinomyces antibioticus ATCC 14570 erhalten werden kann, einem Subfragment davon oder einer dieses enthaltenden DNA-Sequenz, die fähig ist, nach Transformation in einen sensitiven mikrobiellen Wirt eine Resistenz gegenüber TRB zu verleihen;
b) einer Nucleinsäuresequenz, die fähig ist, nach Transformation in einen sensitiven mikrobiellen Wirt eine Resistenz gegenüber TRB zu verleihen, und mit einem vorstehend unter a) erwähnten DNA-Fragment hybridisiert;
c) Teilen von DNA-Insertionen, die mit den unter a) erwähnten Fragmenten hybridisieren und fähig sind, eine TRB-R-vermittelte Resistenz zu verleihen;
d) DNA-Sequenzen, die als Ergebnis der Degeneration des genetischen Codes zu DNA-Sequenzen degeneriert sind, die unter a) oder b) definiert sind und fähig sind, nach Transformation in einen sensitiven mikrobiellen Wirt Resistenz gegenüber TRB zu verleihen,
umfassend:
a) Extraktion der DNA-Fraktion aus dem Genom eines Mikroorganismus, der TRB produziert, und Teilabbau mit einem spezifischen Restriktionsenzym,
b) Ligierung der Restriktionsfragmente in einen Vektor für eine sensitive Wirtszelle,
c) Auswählen der transformierten Zellen mit Hilfe der erworbenen Resistenz und Isolierung der TRB-R-verleihenden Sequenz daraus.

9. Verfahren nach Anspruch 8, wobei der Mikroorganismus Thermoactinomyces antibioticus ATCC 14570 ist.

10. Verfahren nach einem der Ansprüche 8 und 9, wobei das Restriktionsenzym von Schritt a) Sau 3AI ist.

11. Verfahren nach einem der Ansprüche 8 bis 10, wobei das Restriktionsfragment ein DNA-Fragment von etwa 4,0 Kbp ist und der Vektor das BamHI-linearisierte und Phosphatase-erhitzte Plasmid pUC18 ist.

12. Verfahren nach einem der Ansprüche 8 bis 11, wobei die sensitive Wirtszelle E. coli ist.

13. Verwendung einer TRB-R-verleihenden Sequenz nach den Ansprüchen 1, 2, 3 oder 4 zum Nachweis, Isolierung oder Reinigung der entsprechenden Bereiche oder diese enthaltenden Insertionen aus dem Genom einer mikrobiellen Zelle.

14. Verwendung nach Anspruch 13, wobei die mikrobielle Zelle TRB produziert.

15. Verwendung einer TRB-R-verleihenden Sequenz nach Anspruch 1, 2, 3 oder 4 zur Einführung oder Verstärkung der Resistenz eines mikrobiellen Wirts gegenüber TRB.

16. Verwendung nach Anspruch 15, wobei die mikrobielle Zelle TRB produziert.

17. Verfahren zur Isolierung, Identifizierung oder Reinigung einer Nucleinsäuresequenz, die fähig ist, Resistenz gegenüber TRB zu verleihen, oder einer sie enthaltenden Insertion, umfassend die Hybridisierung einer Sonde, die durch eine markierte TRB-R-Sequenz nach den Ansprüchen 1, 2, 3 oder 4 dargestellt ist, mit einer Nucleinsäurefraktion, und gegebenenfalls Isolierung der Nucleinsäurefragmente, die mit der Sonde hybridisieren.

## Revendications

1. Séquence conférant une R-TRB qui comprend une séquence nucléotidique choisie parmi :
a) un fragment d'ADN d'environ 4,0 kpb qui peut être obtenu par traitement par Sau 3AI du génome de Thermoactinomyces antibioticus ATCC 14570, un de ses sous-fragments ou une séquence d'ADN le contenant, qui est capable de conférer une résistance à la TRB par transformation dans un hôte microbien sensible ;
b) une séquence d'acide nucléique qui est capable de conférer une résistance à la TRB par transformation dans un hôte microbien sensible, et de s'hybrider avec un fragment d'ADN mentionné ci-dessus en a) ;
c) des parties d'inserts d'ADN s'hybridant avec les fragments mentionnés en a) et capables de conférer une résistance à médiation par la R-TRB ;
d) des séquences d'ADN qui sont dégénérées par suite du code génétique dégénéré en séquences d'ADN définies en a) ou b) et sont capables de conférer une résistance à la TRB par transformation dans un hôte microbien sensible.

2. Séquence conférant la R-TRB qui est un fragment d'ADN d'environ 4,0 kpb pouvant être obtenu par traitement par Sau 3AI du génome de Thermoactinomyces antibioticus ATCC 14570, un sous-fragment de celui-ci ou une séquence d'ADN le contenant, qui est capable de conférer une résistance à la TRB par transformation dans un hôte microbien sensible.

3. Séquence conférant la R-TRB qui est une séquence d'acide nucléique capable de conférer une résistance à la TRB par introduction dans un hôte microbien sensible et s'hybride avec un fragment d'ADN d'environ 4,0 Kpb qui peut être obtenu par traitement par Sau 3AI du génome de Thermoactinomyces antibioticus ATCC 14570, un sous-fragment de celui-ci ou une séquence d'ADN le contenant.

4. Séquence conférant la R-TRB qui est une portion d'un insert d'ADN s'hybridant avec un fragment d'ADN d'environ 4,0 Kpb pouvant être obtenu par traitement par Sau 3AI du génome de Thermoactinomyces antibioticus ATCC 14570, un sous-fragment de celui-ci ou une séquence d'ADN le contenant, qui est capable de conférer une résistance à la TRB par transformation dans un hôte microbien sensible.

5. Plasmide recombinant convenant pour la transformation d'un hôte microbien comprenant une séquence conférant une R-TRB selon les revendications 1, 2, 3 ou 4.

6. Cellule hôte microbienne transformée par un plasmide recombinant selon la revendication 5.

7. Cellule microbienne selon la revendication 6, qui est productrice de thermorubine.

8. Procédé pour préparer une séquence conférant la R-TRB choisie parmi :
a) un fragment d'ADN d'environ 4,0 kpb qui peut être obtenu par traitement par Sau 3AI du génome de Thermoactinomyces antibioticus ATCC 14570, un de ses sous-fragments ou une séquence d'ADN le contenant, qui est capable de conférer une résistance à la TRB par transformation dans un hôte microbien sensible ;
b) une séquence d'acide nucléique qui est capable de conférer une résistance à la TRB par transformation dans un hôte microbien sensible, et s'hybride avec on fragment d'ADN mentionné ci-dessus en a) ;
c) des parties d'inserts d'ADN s'hybridant avec les fragments mentionnés en a) et capables de conférer une résistance à médiation par R-TRB ;
d) des séquences d'ADN qui sont dégénérées par suite du code génétique dégénéré en séquences d'ADN définies en a) ou b) et sont capables de conférer une résistance à la TRB par transformation dans un hôte microbien sensible ;
qui comprend :
a) le fait d'extraire la fraction d'ADN du génome d'un micro-organisme qui est producteur de TRB et de le digérer partiellement avec une enzyme de restriction spécifique ;
b) le fait de ligaturer les fragments de restriction à un vecteur pour une cellule hôte sensible ;
c) le fait de sélectionner les cellules transformées au moyen de la résistance acquise et d'isoler de celles-ci la séquence conférant la R-TRB.

9. Procédé selon la revendication 8, dans lequel le micro-organisme est Thermoactinomyces antibioticus ATCC 14570.

10. Procédé selon l'une quelconque des revendications 8 et 9, dans lequel l'enzyme de restriction de l'étape a) est Sau 3AI.

11. Procédé selon l'une quelconque des revendications 8 à 10, dans lequel le fragment de restriction est un fragment d'ADN d'environ 4,0 Kpb et le vecteur est le plasmide pUC18 linéarisé par BamHI et traité par la phosphatase.

12. Procédé selon l'une quelconque des revendications 8 à 11, dans lequel la cellule hôte sensible est E. coli.

13. Utilisation d'une séquence conférant la R-TRB selon les revendications 1, 2, 3 ou 4 pour détecter, isoler ou purifier les régions correspondantes, ou des inserts les contenant, à partir du génome d'une cellule microbienne.

14. Utilisation selon la revendication 13, dans laquelle la cellule microbienne est un producteur de TRB.

15. Utilisation d'une séquence conférant la R-TRB selon les revendications 1, 2, 3 ou 4 pour introduire ou augmenter la résistance d'un hôte microbien à la TRB.

16. Utilisation selon la revendication 15, dans laquelle la cellule microbienne est un producteur de TRB.

17. Procédé pour isoler, identifier ou purifier une séquence nucléique capable de conférer une résistance à la TRB ou un insert la contenant, qui comprend l'hybridation d'une sonde représentée par une séquence R-TRB marquée selon les revendications 1, 2, 3 ou 4, avec une fraction d'acide nucléique et éventuellement l'isolement des fragments d'acide nucléique qui s'hybrident avec la sonde.
